(19) Europäisches Patentamt
European Patent Office
Office européen des brevets

(11) Publication number : **0 476 875 A2**

(12) **EUROPEAN PATENT APPLICATION**

(21) Application number : **91307994.3**

(51) Int. Cl.[5] : **B01D 61/02,** B01D 71/42

(22) Date of filing : **30.08.91**

(30) Priority : **02.09.90 IL 95561**

(43) Date of publication of application :
**25.03.92 Bulletin 92/13**

(84) Designated Contracting States :
**AT BE CH DE DK ES FR GB GR IT LI LU NL SE**

(71) Applicant : **MEMBRANE PRODUCTS KIRYAT WEIZMANN LTD.**
**POB 138 Kiryat Weizmann**
**Rehovot (IL)**

(72) Inventor : **Linder, Charles**
**20 Derech Yavne**
**Rehovot (IL)**
Inventor : **Perry, Mordechai**
**4 Kaf Zayin Benissan Street**
**Petach Tikva (IL)**
Inventor : **Nemas, Mara**
**28/15 Tamar Street**
**Neve Monosson (IL)**

(74) Representative : **Hillier, Peter et al**
**Reginald W. Barker & Co., 13, Charterhouse Square**
**London, EC1M 6BA (GB)**

(54) **Process for the purification and concentration of biologically active materials.**

(57) Biologically active substances having a molecular weight below 1500, present in an initial admixture with organic solvent media and their mixtures with water, are purified and/or concentrated by a method comprises applying the initial admixture under superatmospheric pressure to the feed surface of a solvent stable membrane which is additionally characterized by the fact that it possesses simultaneously properties (i) and (ii), namely, (i) rejecting at least part of such substance in the initial admixture, and (ii) allowing the permeation of at least part of said the medium, the balance of properties (i) and (ii) being such that there accumulates at the membrane feed surface the biologically active substance(s) in admixture with at least part of the medium, in a concentration which is higher than that in the initial admixture.

EP 0 476 875 A2

## FIELD OF THE INVENTION

The present invention relates to a method for the purification and concentration purification of biologically active materials, by means of membranes.

## BACKGROUND OF THE INVENTION

Biologically active materials such as antibiotics and peptides are frequently prepared and purified in organic solvent media or mixtures of such media with water. The measures necessary to concentrate and purify such materials are often slow and costly, and moreover must be used with extreme care in order to prevent decomposition and loss of activity of such materials in the course of processing. The use of high boiling solvents such as dimethylformamide (DMF), dimethylacetamide (DMAC), dimethylsulfoxide (DMSO) and N-methylpyrrolidone (NMP) presents particular difficulties in this respect.

## SUMMARY OF THE INVENTION

The present invention provides a method for purifying and/or increasing the concentration of at least one substance selected from biologically active substances having a molecular weight below 1500, present in an initial admixture with organic solvent media or their mixtures with water, which method comprises the procedure of applying the initial admixture under superatmospheric pressure to one surface, designated the feed surface, of a solvent stable membrane which is additionally characterized by the fact that it possesses simultaneously properties (i) and (ii), namely, (i) rejecting at least part of the at least one substance when present in the initial admixture, and (ii) allowing the permeation of at least part of the at least one medium, wherein the balance of properties (i) and (ii) is such that there accumulates at the feed surface the at least one substance in admixture with at least part of the at least one medium, in a concentration which is higher than that in the initial admixture.

It is also within the scope of the present invention to periodically monitor the concentration of the substance at the feed surface, and to terminate the procedure when a predetermined increase in the concentration, compared with concentration in the initial admixture, has been achieved.

Stable solvent membranes which are suitable for carrying out the method of the present invention have been described in our Israel Patent Application No. 89970 as originally filed April 14, 1989, and in U.S. Patent Application Serial Nos. 07/416,224 and 07/415,156, both filed October 2, 1989. The entire disclosures of the foregoing patent applications are incorporated herein by reference.

## DETAILED DESCRIPTION OF THE INVENTION

According to a particular embodiment of the invention, the substances which may be purified/or concentrated in accordance with the method of the invention may be e.g. enzymes, hormones, proteins, antibodies, α-aminoacids, dipeptides, tripeptides, tetrapeptides and higher order peptides. Exemplary peptides are Glu-Ala-Glu, Glu-Ala-Glu-Asn, Lys-Glu-Glu-Ala-Glu, Val-Glu-Glu-Ala-Glu, kentsin, tuftsin; Glu-His-Gly amide, Gly-Arg-Gly-Asp, diprotin, casomorphin, aspartame, and functional derivatives of any of the foregoing, and their salts and addition compounds, and mixtures thereof.

Alternatively, in accordance with another embodiment of the invention, the substances which may be purified/or concentrated in accordance with the method of the invention may be e.g. penicillins, cephalosporins, tetracyclines, erythromycins, chloramphenicols, sulfonamide antibacterials, other antibacterials, streptomycin, gentamycin, other aminoglycosidic antibiotics, other antibiotics, and functional derivatives of any of the foregoing, and their salts and addition compounds, and mixtures thereof. By way of example only, of such a mixture to which the inventive method may be applied, there may be mentioned trimethoprim-sulfomethoxazole mixtures, which are the subject of commercial manufacture.

The solvent stable membrane which is utilized in accordance with the method of the invention is preferably made from (but not restricted to) a polymer which is selected from copolymers and homopolymers of ethylenically unsaturated nitriles, which has been subjected to a post-polymerization treatment (in order to impart to the membrane the desired characteristics), e.g. such treatment which includes at least one of the following steps, namely: cross-linking; treatment with a diazonium salt; and/or coating with a further polymer.

According to a particular embodiment, the solvent stable membrane comprises a crosslinked layer of less than 5 microns (preferably less than 1 micron) in thickness, which layer includes at least one polymer selected from polyphenylene oxide type polymers and polysulfone type polymers, supported on a solvent stable porous membrane substrate. The crosslinked layer is optionally ionically charged.

Thus, by way of example only, the layer may include or be constituted from a bromomethylated 2,6-dimethylphenylene oxide polymer (i.e. 2,6-dimethylphenylene oxide polymer in which some of the methyl groups have been converted to bromomethyl, by bromination), which has been crosslinked (e.g. with a diamine such as m-phenylenediamine) and which has been subsequently derivatized, particularly in order to influence flux and rejection properties of the final membrane, e.g. by quaternization and/or sulfonation, so as to introduce polar, e.g. ionic groups, and/or to introduce hydrophobic groups.

The substrate may be selected from, for example, microfiltration membranes and ultrafiltration membranes. More particularly, the substrate may include at least one material selected from crosslinked polyacrylonitrile, polyarylene oxides, polyarylene sulfones, polyethylene, polypropylene, other polyolefins, tetrafluoropolyethylene, other perfluoro polymers, aromatic polyimides, glass, ceramics and carbon.

It will be appreciated that solvent stable membranes other than those specifically mentioned herein may be utilized in the method of the present invention, provided that they reject molecules having a molecular weight below 1500 Daltons.

In a particular embodiment, the solvent stable membrane utilized in accordance with the method of the invention may be made by a process which includes applying the following sequential steps to a porous membrane substrate cast from a polymer, selected from copolymers and homopolymers of ethylenically unsaturated nitriles, namely:

(a) cross-linking the porous membrane substrate;
(b) treating the cross-linked substrate with a diazonium salt;
(c) treating the product of step (b) with alkali;
(d) treating the product of step (c) with a compound which is at least difunctional;
(e) coating the product of step (d) with a hydrophilic polymer; and
(f) subjecting the product of step (e) to the action of a cross-linking compound which is at least difunctional.

It may be noted that the diazonium salt in step (b) may be either monomeric or polymeric, the monomeric or polymeric diazonium salt thus being physically or electrostatically adsorbed; and that the effect of step (c) is believed to eliminate the diazonium groups with the formation of cross-links with the porous membrane substrate, but leaving reactive groups capable of reacting with the at least difunctional compound in step (d) and/or the hydrophilic polymer in step (e). Step (d) moreover provides additional reactive sites for chemical reaction with the hydrophilic polymer in step (e). For applications to solutions comprising polar solvents, it is preferred that at least one of the hydrophilic polymer in step (e) and the cross-linking compound in step (f) contains ionic groups; in this preferred embodiment, the hydrophilic polymer coating of step (e) is thus charged ionically, as well as being cross-linked. In the case of applications with hydrophobic non-polar solvents, it is preferred that the outer layer of the membrane include hydrophobic, e.g. alkyl or aromatic groups; such hydrophobic groups may be contained as derivatized pendents on the hydrophilic polymer of step (e), or in the cross-linking compound used in step (f).

The initial porous membrane substrate, which is cross-linked in step (a), may be e.g. a reverse osmosis (RO), ultrafiltration (UF) or microfiltration (MF) membrane with an average pore size in the range of from 1 to 500 nm, preferably 1 to 100 nm, and most preferably 2 to 20 nm. In addition, a minimum porosity of about 10% is presently preferred, in order to achieve a sufficiently high flux.

The copolymers and homopolymers of ethylenically unsaturated nitriles referred to herein may be, for example, copolymers or homopolymers of acrylonitrile, ($C_{1-6}$-alkyl)acrylonitriles such as methacrylonitrile and hexylacrylonitrile, arylacrylonitriles such as (optionally substituted) phenylacrylonitrile, halo-substituted acrylonitriles such as fluoroacrylonitrile and chloroacrylonitrile and bromoacrylonitrile, as well as other substituted acrylonitriles such as thioloacrylonitrile. Homopolymers of acrylonitrile are presently preferred.

In the case of copolymers, suitable comonomers for copolymerization with the ethylenically unsaturated nitriles may contain, e.g., hydrophobic, hydrophilic, polar or ionic groups. Exemplary comonomers are vinyl esters of 2-18 carboxylic acids such as vinyl acetate; vinylpyridine; vinyl chloride; styrene; acrylic and methacrylic acids and their esters with e.g. $C_{1-4}$ alcohols; maleic anhydride; 2-aminoethyl methacrylate; allyl compounds such as allyl alcohol, allyl- and methallyl- sulfonic acids and their salts, e.g. the alkali metal salts, allyl and methallyl halides, allylamine and allyl p-toluenesulfonate. The term "copolymers" herein includes copolymerization products of ethylenically unsaturated nitriles with one or more comonomers; this term thus includes, by way of example acrylonitrile/styrene/ butadiene (ABS), acrylonitrile/methyl methacrylate/vinyl acetate, and acrylonitrile/methyl methacrylate/sodium allylsulfonate terpolymers, and quadripolymers. In the copolymers, the proportion of ethylenically unsaturated nitrile(s) by weight is preferably at least 20%, more preferably at least 50%, and most preferably at least 80% of the total monomers.

The initial porous membrane substrate may be cast by any of the methods known in the art, see e.g., US 4,902,422, US 4,029,582, GB 2,000,720A, US 3,556,305, US 3,615,024 and US 3,567,810, the entire contents of all these disclosures being deemed to be incorporated herein by reference. Thus, for example, a suitable

polymer may be dissolved in a solvent or solvent mixture (including, e.g., NMP, DMF, DMSO, DMAC, hexamethylphosphotriamide and/or dioxane), optionally containing one or more of the following additives, namely, co-solvents, non-solvents, salts, other electrolytes, surfactants, for altering or modifying the membrane morphology and its flux and rejection properties. A non-limiting list of such additives includes acetone, ethanol, methanol, formamide, water, methyl ethyl ketone, triethyl phosphate, sulfuric acid, hydrochloric acid, partial esters of fatty acids and sugar-derived alcohols as well as their ethylene oxide adducts, sodium dodecyl sulfate, sodium dodecyl sulfonate, NaOH, KCl, $ZnCl_2$, $CaCl_2$, $LiNO_3$, LiCl and $Mg(ClO_4)_2$.

For purposes of definition, it is to be understood that the initial admixture to which the method of the invention is applied, is not merely a mixture from which the biologically active substance can be separated by conventional filtration techniques, but with this reservation it may be a solution, dispersion, suspension or any other type of admixture. The at least one medium may include (by way of non-limiting examples) a high-boiling solvent selected from dimethylformamide, dimethylacetamide, dimethylsulfoxide and N-methylpyrrolidone. By way of additional exemplification, the medium may include, alternatively or additionally, either low or high boiling solvents, such as a solvent selected from aliphatic alcohols containing 1 to 6 carbon atoms, alkyl acetates having from 1 to 6 carbon atoms in the alkyl group, aliphaticethers containing 4 to 8 carbon atoms, aliphatic ketones containing 3 to 8 carbon atoms, aliphatic nitriles containing 1 to 6 carbon atoms, aliphatic hydrocarbons containing from 5 to 10 carbon atoms, halohydrocarbons and aromatic hydrocarbons.

It will be evident to persons skilled in the art that the membranes themselves may have any convenient configuration, such as flat sheets, tubes, tubelets, hollow fibers or they may be spiral-wound.

It will be appreciated that a particular advantage of the present invention is that, in order to avoid decomposing and deactivating heat-sensitive biologically active substances to which the inventive method is applied, the method may be conducted at ambient temperature.

In accordance with the method of the present invention, the concentration of the biologically active substance in the initial admixture is increased by virtue of the fact that the utilized membrane rejects the substance in question and permits permeation of the solvent. The degree of achievable concentration may be up to the saturation point of the solute in the solvent, or even beyond this point. Persons skilled in the art will be aware that, notwithstanding that the objective of the inventive method is an increase in the concentration of the biologically active substance, the initial admixture may nevertheless be diluted in order to give a higher initial flux or otherwise increase the rejection of the membrane for the desired solute. Moreover, the admixture at any time during the performance of the inventive method may be similarly diluted in order to give a higher initial flux or otherwise increase the rejection of the membrane for the desired solute, or even to increase the solubility of the solute (as its concentration increases) in the at least partially exchanged solvent. Addition of solvent during operation of the inventive method, which may be continuous or intermittent, may be optionally such as to maintain a constant volume of solvent as the concentration of the desired solute increases. After application of the method of the invention, the obtained solute/solvent admixture may of course be subjected to such known processes as chromatography, electrodialysis, crystallization, lyophilization, evaporation, precipitation, selective adsorption, and/or any other processes used in the art for retrieving and purifying materials.

It will further be appreciated by skilled persons that low molecular weight components (such as, for example, low molecular weight salts such as NaCl) which are present in the initial admixture may be simultaneously separated from the desired solute as the concentration of the latter increases, by permeation through the membrane.

In general terms, a membrane which has a cutoff at 1500 MW for a neutral molecule will reject substances of MW above about 1500 and allow the permeation of substances of MW below this figure. For charged molecules, however, the same membrane may effectively reject molecules of 300 MW. Similarly, a membrane which has a cutoff at 300 MW for neutral molecules will reject substances of MW above about 300 and allow the permeation of substances of MW below about 300, and a membrane which has a cutoff at 150 MW will reject substances of MW above about 150 and allow the permeation of substances of MW below about 150. In most cases, it is found that charged molecules are more effectively rejected and that the cutoff for charged molecules is different from that for neutral molecules, when using the same membrane. It will be evident from this discussion that a membrane can be selected with a particular cut off in order to retain particular substances and allow the permeation of others, which are present in the initial admixture.

The separation effect (rejection) of the membranes useful according to the present invention can be measured as follows. A circular membrane with a surface area of 13 $cm.^2$, resting on a sintered stainless steel disc, is used in a cylindrical cell made of stainless steel. 150 ml. of the solution (to be tested) which contains the substance to be tested in a concentration $C^1$ (g. substance/g. solution) are introduced onto the membrane in the steel cylinder and subjected to a pressure ($N_2$) of 14 bars. The solution is stirred magnetically. The liquid which collects on the outlet side of the membrane is examined to determine the concentration ($C^2$) therein of the substance to be tested, 3 samples of 5 ml. each being taken from the start of the experiment. In general,

the amount which flows through the membrane and the composition of the 3 samples are constant. The rejection (R) can be calculated from the values obtained, using the equation:

$$R(\%) = (C^1 - C^2) \times 100/C^1.$$

The amount of material (F = flux) passing through the membrane per surface and time unit is determined from the equation:

$$F = V \times S^{-1} \times t^{-1}$$

where V = volume, S = membrane surface area and t = time. F may be expressed in terms of cubic meters of solution per square meter surface area of membrane per day, or in terms of liters of solution per square meter surface area of membrane per hour.

In addition to measurements on flat membranes, measurements on tubular membranes 60 cm. long and with an outer diameter of 1.4 cm. were also carried out. For this purpose the tubular membranes were placed in a perforated tube made of stainless steel. The whole (i.e. membrane with support) is then placed in a tube made of polycarbonate. The outflow from the membrane is between this outer polycarbonate tube and the steel tube. The liquid is added as a stream of solution in turbulent or laminar flow under pressure. The flow rate is kept constant at 10-15 liters per minute. The rejection (R) and the flux (F) are calculated in the same way as for flat membranes.

The solvent stable membrane for performing the method according to the invention may be, for example, one of those described in paragraphs (A), (B), (C) and (D), below.

(A) The solvent stable membrane may comprise a porous substrate, such as a substrate cast from a polymer, selected from copolymers and homopolymers of ethylenically unsaturated nitriles, which substrate has been coated with a silicone layer. Thin crosslinked polysiloxane films on porous supports have been described (for purposes different from that of the present invention) in U.S. Patents Nos. 4,230,463, 4,243,701 and 4,553,983, the entire contents of all these patents being incorporated herein by reference.

(B) The solvent stable membrane may comprise a porous substrate, such as a substrate cast from a polymer, selected from copolymers and homopolymers of ethylenically unsaturated nitriles, which substrate has first been treated with a pore protector in absence of a curing agent, and the pore-protected substrate has then been coated with a silicone layer. The product is the subject of our copending patent application no.(13219), the entire contents of which are incorporated herein by reference. The pore protector, which may be, for example, a hydroxy-terminated polysiloxane (used for this purpose in absence of curing agents or catalysts), serves the dual purpose of preventing the pores from collapsing, when the support is dried during the curing of the silicone layer, and of preventing passage of the coating material deeply into the pores and thus also preventing an undue reduction of the flux of the finished coated membrane. Treatment with the pore protector may be carried out, for example, by dipping the membrane into a dilute solution of the pore protector in a low-boiling inert solvent, e.g. a low boiling alcohol such as methanol, ethanol, propanol or butanol. It may be noted in passing that the use of a methyl terminated polysiloxane as drying agent for cellulose acetate has been described in U.S. Patent No. 4,855,048, the contents of which are incorporated herein by reference.

(C) The solvent stable membrane may be made by a process which includes applying the following sequential steps to a porous membrane substrate which has been crosslinked and insolubilized, (e.g., from a polymer selected from copolymers and homopolymers of ethylenically unsaturated nitriles, which has been crosslinked and insolubilized, particularly such substrate derived from polyacrylonitrile which has been heated in a basic medium at an elevated temperature), namely: (a) applying to the surface and pores of the substrate, a coating of reactive polymer, such as a polyamine such as polyethyleneimine; (b) treating the cross-linked coated substrate with a multifunctional agent such as a poly-$\alpha$-imidohalide (i.e. containing more than one -C(:NH)-X group where X is halogen), e.g., cyanuric chloride or tetrachloropyrimidine; (c) treating the product of step (b) with further reactive polymer which may be the same as or different from that mentioned in step (a); and (d) treating the product of step (c) with further multifunctional agent which may be the same as or different from that mentioned in step (b). In step (d), the further reactive polymer applied in step (c) is crosslinked and the hydrophobic/hydrophilic balance may be adjusted simultaneously. The reactive polymer(s) and/or multifunctional agent(s) may contain ionic or hydrophobic groups as well as reactive functions, in order to adjust the hydrophobic/hydrophilic balance, and thus the flux, as required. The membrane product may accordingly be tailored to the intended use; for example, these groups may be ionic (e.g. sulfonic, carboxylic, quaternary ammonium, hydroxyl amines) in the case of use with polar solvents, or hydrophobic (e.g. aromatic or alkyl groups) in the case of use with nonpolar solvents. Examples of crosslinking agents are described in U.S. Patents Nos. 4,690,765, 4,690,766 and 4,778,596, the entire contents of all these patents being incorporated herein by reference.

(D) The solvent stable membrane comprises a crosslinked layer of less than 5 microns, preferably less than 1 micron, in thickness, which layer includes at least one polymer selected from polyphenylene oxide type

polymers and polysulfone type polymers (provided that the crosslinked layer includes at least one polymer derived from halomethylated, e.g. chloromethylated or bromomethylated, polyphenylene oxide type monomers), supported on a solvent stable porous membrane substrate (such as a substrate cast from a polymer, selected from copolymers and homopolymers of ethylenically unsaturated nitriles, particularly polyacrylonitrile), wherein the crosslinkers utilized for crosslinking the polyphenylene oxide type polymeric layer are selected from aliphatic, cycloaliphatic, araliphatic and aromatic amines, which may be monoamines and/or polyamines, such as mono and/or poly primary amines. The product is the subject of our copending patent application no.(13218), the entire contents of which are incorporated herein by reference. Suitable amine crosslinkers are described, for example, in U.S. Patents Nos. 4,468,500, 4,468,501, and 4,468,502, and in EP 0,130,963A1, the entire contents of all these patent documents being incorporated herein by reference. The crosslinked layer may be optionally ionically charged. The crosslinking operation is preferably carried out after the said layer has been applied and dried. Thus, for example, the coated membrane may be immersed in a solution (e.g. an aqueous solution, optionally containing small proportions of organic solvent/s to swell the layer and thus facilitate the reaction) of crosslinker(s). It should be noted that the substrate is not restricted to one prepared from copolymers and homopolymers of ethylenically unsaturated nitriles, but that other substrate materials, such as those described elsewhere herein, may be used.

The following optional conditions may be applied to the solvent stable membranes (A), (B) and (C), namely: the substrate may be selected from microfiltration membranes and ultrafiltration membranes and/or it may include at least one material selected from crosslinked polyacrylonitrile, polyarylene oxides, polyarylene sulfones, polyethylene, polypropylene, other polyolefins, tetrafluoropolyethylene, other perfluoro polymers, aromatic polyimides, glass, ceramics and carbon.

In the non-limiting Examples which follow, the compounds of formulae (1) to (6) (see below) may be used as reactive agents for cross-linking and charging the second polymer layer, unless otherwise indicated. Nonionic crosslinkers such as carbonic acid amy be used additionally or alternatively.

## EXAMPLE I

Polyacrylonitrile (98% homopolymer: DuPont A), MW (number average) 50,000, was dissolved in dimethylformamide (DMF), bob cast onto a tube of nonwoven polyester (1.27 cm. diameter) and gelled in ice-water. After washing with water for 12 hours, this substrate membrane had a 65% rejection to polyethylene glycol of 60K MW, and 5% rejection to sucrose. The polyacrylonitrile substrate was crosslinked by immersion for 15 minutes in 1% wt./vol. ethanolic sodium ethoxide, drained and then heated to 115°C for 30 minutes. The original substrate was off-white or beige, but after this treatment, it was dark brown, and no longer soluble or swellable in DMF, NMP or DMSO. While the original membrane had less than 18% rejection to raffinose, the crosslinked membrane had a rejection of 45% to raffinose and a 94% rejection to dextran 70K. The crosslinked membrane was subsequently modified by the following procedure. It was immersed for 10 minutes in a 0.5% aqueous diazonium solution (p-phenylenediamine + sodium nitrite + HCl), then in 2% aqueous sodium hydroxide solution for 10 minutes, washed 30 minutes with tap water, crosslinked with cyanuric chloride (2% aqueous suspension) at pH 9 and 0°C for 5 minutes, washed with water for 30 minutes and treated with 8% aqueous polyethyleneimine solution at pH 10 for 10 minutes, washed with water for 2 hours, treated with an aqueous solution containing 1% of the reactive dye of formula (1), and 10% NaCl for 15 minutes, immersed in a solution of 2% sodium carbonate for 30 minutes, immersed in 2% cyanuric chloride at pH 9.0 for 10 minutes, washed well with tap water for 30 minutes and immersed in sodium hydroxide solution at pH 10 for 12 hours. The resultant composite membrane had a 99% rejection to dextran 2000 MW, 97% to glucose and 99.9% (before modification, 50%) rejection to Congo Red, with fluxes of 2000 $l/m^2$.d to water.

The product membrane had a rejection to aspartame in water of 99.5%, and to aspartame in 25% acetonitrile/75% water of 98.5%. The aspartame was concentrated from 500 ppm to 50,000 ppm. The product membrane moreover had a rejection to Penicillin G in 50% aqueous DMF solution of 99%; Penicillin G could be concentrated from 1000 ppm to 10,000 ppm. The starting and final flux were respectively 500 $l./m^2$.d and 150 $l./m^2$.d. The applied pressure was 40 atm. at 20°C.

(1)

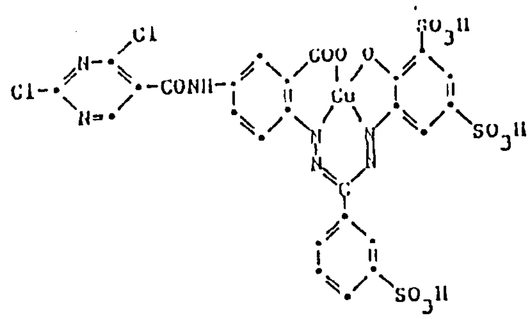

(2)

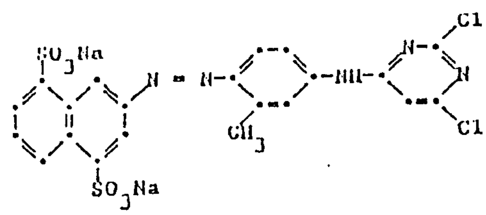

(3)

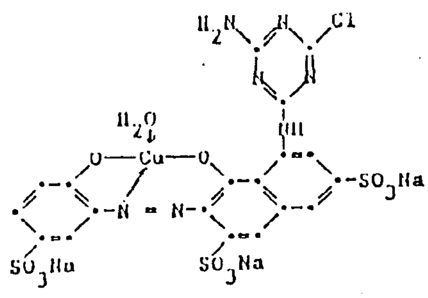

(4)

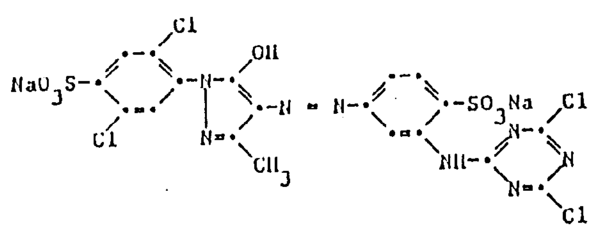

(5)

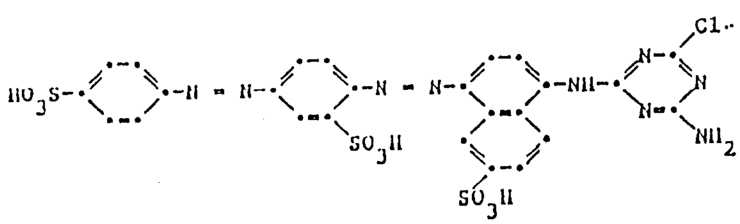

(6) 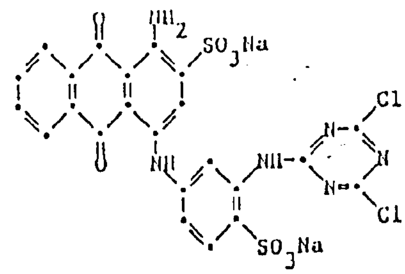

EXAMPLE II

Crosslinked polyacrylonitrile substrate (unmodified) prepared as described in Example I was treated with a 10% aqueous solution of sodium hydroxide at 50°C for 15 minutes, washed well with tap water, and heated (e.g. in air, steam, or water, or in a high boiling solvent such as glycerol) for 15 minutes at 110°C and stored in water. The membrane was immersed for 10 minutes in a 0.5% aqueous diazonium solution (p-phenylenediamine + sodium nitrite + HCl), then in 2% aqueous sodium hydroxide solution for 10 minutes, washed 30 minutes with tap water, crosslinked with cyanuric chloride (2% aqueous suspension) at pH 9 and 4°C for 5 minutes, washed 30 minutes with tap water and treated with 8% aqueous polyethyleneimine solution at pH 10 for 10 minutes, washed with tap water for 2 hours, treated with a solution containing 1% of the reactive dye of formula (1) and 10% NaCl for 15 minutes, immersed in an aqueous solution of 2% sodium carbonate for 30 minutes, washed well with tap water for 30 minutes and immersed in sodium hydroxide solution at pH 10 for 12 hours. The unmodified membrane had a 70% rejection to Congo Red, but after modification, more than 99%. The performance of this membrane is similar to that of Example 1, for the same solute/solvent combinations. The stability of these membranes is shown in Table 1 for common but aggressive polymer solvents. The results show a uniformly high rejection after the tests.

Table 1: Stability to Solvents of the Membrane of Example II.

| Run No. | Solvent♦ | Immersion Time (hours) | Flux($l/m^2$.d) at ~30 bar | Rejection to Penicillin G* |
|---|---|---|---|---|
| 1 | water | control | 1860 | 99 |
| 2 | DMF | 170 | 982 | 98 |
| 3 | NaOH 4% (pH 14) | 24 | 1479 | 62 |
| 4 | water | control | 2361 | 99 |
| 5 | water (100°C) | 24 | 2000 | 93 |
| 6 | **MEK | 24 | 915 | 91 |
| 7 | $CH_2Cl_2$ | 24 | 647 | 99 |
| 8 | water | control | 1231 | 97 |
| 9 | HCl 1.3% (pH 0.5) | 80 | 361 | 98 |
| 10 | NaOH 0.04% (pH 12) | 80 | 472 | 96 |
| 11 | DMF | 80 | 1189 | 92 |

♦room temperature unless otherwise specified
*1000 ppm in 50/50 aq. acetonitrile **MEK = methyl ethyl ketone

A 50/50 aqueous acetonitrile solution of 500 ppm aspartame, containing also 2000 ppm NaCl was concentrated, and the NaCl content reduced by continually adding 50/50 aqueous acetonitrile in the test procedure to maintain a constant volume, while utilizing the product membrane of Example II. After the addition of 2 x the original volume, addition of solvent was stopped and the aspartame was found to be concentrated to 2500 ppm with an approximately 50% reduction in the NaCl content.

EXAMPLE III

A mixture of 1% bromomethylated 2,6-dimethylphenylene oxide (MW 20,000, 3.2 meq./g. of active Br) and 0.2% m-phenylenediamine in toluene is used to coat a 20 microns thick microporous polypropylene (30% porosity) containing 0.45 x 0.045 micron rectangular pores, to form a 0.5 micron layer. This composite is then quaternized with trimethylamine. The resultant membrane had a 94% rejection to 500 ppm aspartame in 25:75 acetonitrile/water, with a flux of 400 $1./m^2$.d.

EXAMPLE IV

The insolubilized support of Example II is immersed for 10 minutes in a 1% aqueous polyethyleneimine (MW 30,000) solution at 8.5, washed well with tap water for 15 minutes, immersed in a 2% suspension of cyanuric chloride at pH 9.0 and 4°C for 5 minutes, washed well with tap water for 10 minutes, immersed for 15 minutes in an 8% aqueous polyethyleneimine (MW 30,000) solution at 8.5, washed well with tap water for 2 hours, immersed in a 2% suspension of cyanuric chloride at pH 9.0 and 4°C for 5 minutes, and washed well

with 2% aqueous sodium carbonate (15 minutes), followed by tap water. The resultant membrane had a 99% rejection to glucose in water, and a 98% rejection to 500 ppm aspartame in 50:50 acetonitrile/water, with a flux of 400 l./m$^2$.d.

EXAMPLE V

The insolubilized support of Example II is coated with a toluene solution of 1% bromomethylated 2,6-dimethylphenylene oxide as in Example III (omitting the m-phenylenediamine), forming a 0.75 micron thick layer. After drying, the composite is immersed in a 10% aqueous ammonium hydroxide solution for 2 hours, and after removal is well rinsed with tap water. The PPO coating was thus crosslinked by the ammonia. The resultant membrane had a 99% rejection to 10% erythromycin in ethyl acetate, with a flux of 400 l./m$^2$.d.

EXAMPLE VI

The insolubilized membrane of Example II is immersed in 0.5% silanol-terminated polydimethylsiloxane (MW 4200) in isopropanol for 5 minutes, drained, heated at 50°C for 30 minutes, immersed for 5 minutes in a solution in hexane containing 0.5% silanol-terminated polydimethylsiloxane (MW 36,000), 0.8% tetraethyl silicate and 2% tin octoate, and drained at room temperature for 72 hours. The resultant membrane had a 99% rejection to 10% erythromycin in ethyl acetate, with a flux of 950 l./m$^2$.d. When the treatment with silanol-terminated polydimethylsiloxane, MW 4200 (the pore protector) was omitted, the product membrane had a rejection to 10% erythromycin in ethyl acetate of 90% and a flux under these conditions of 2500 l/m$^2$.d.

While the present invention has been particularly described herein, persons skilled in the art will appreciate that many modifications and variations may be made in the practice of the invention. Accordingly, the invention is not to be construed as limited to the embodiments which have been particularly described, rather its concept, spirit and scope may be more readily understood from a consideration of the claims which follow.

## Claims

1. A method for treating at least one substance selected from biologically active substances having a molecular weight below 1500, present in an initial admixture with at least one medium selected from the group consisting of organic solvent media and their mixtures with water, for the purpose of achieving art least one of the following objectives, namely:
   - purifying said at least one substance, and/or
   - increasing the concentration of said at least one substance in said initial admixture,

   which method comprises the procedure of applying said initial admixture under superatmospheric pressure to one surface, designated the feed surface, of a solvent stable membrane which is additionally characterized by the fact that it possesses simultaneously properties (i) and (ii), namely, (i) rejecting at least part of said at least one substance when present in said initial admixture, and (ii) allowing the permeation of at least part of said at least one medium, wherein the balance of properties (i) and (ii) is such that there accumulates at said feed surface said at least one substance in admixture with at least part of said at least one medium, in a concentration which is higher than that in the said initial admixture.

2. A method according to claim 1, wherein said solvent stable membrane is made from a polymer, selected from copolymers and homopolymers of ethylenically unsaturated nitriles, which has been subjected to a post-polymerization treatment in order to impart to the membrane the desired characteristics, and wherein said post-polymerization treatment preferably includes least one of the following steps, namely: cross-linking; treatment with a diazonium salt; and coating with a further polymer.

3. A method according to claim 2, wherein said solvent stable membrane is made by a process which includes applying the following sequential steps to a porous membrane substrate cast from a polymer, selected from copolymers and homopolymers of ethylenically unsaturated nitriles, namely: (a) cross-linking said porous membrane substrate; (b) treating the cross-linked substrate with a diazonium salt; (c) treating the product of step (b) with alkali; (d) treating the product of step (c) with a compound which is at least difunctional; (e) coating the product of step (d) with a hydrophilic polymer; and (f) subjecting the product of step (e) to the action of a cross-linking compound which is at least difunctional and which preferably contains ionic groups.

4. A method according to either claim 1 or claim 2, wherein the solvent stable membrane comprises a crosslinked layer of less than 5 microns in thickness, which layer includes at least one polymer selected from polyphenylene oxide type polymers and polysulfone type polymers, supported on a solvent stable porous membrane substrate.

5. A method according to claim 4, wherein at least one of the following conditions applies, namely:
– said crosslinked layer is ionically charged;
– said crosslinked layer is of less than 1 micron in thickness;
– said substrate is selected from microfiltration membranes and ultrafiltration membranes;
– said crosslinked layer includes a crosslinked bromomethylated 2,6-dimethylphenylene oxide polymer which has been derivatized so as to introduce ionic groups;
– said substrate includes at least one material selected from crosslinked polyacrylonitrile, polyethylene, polypropylene, other polyolefins, aromatic polyimides, glass, ceramics and carbon.

6. A method according to any of the preceding claims, wherein at least one of the following conditions applies, namely:
– said at least one substance is selected from the group consisting of $\alpha$-aminoacids, dipeptides, tripeptides, tetrapeptides and higher order peptides;
– said at least one substance is selected from the group consisting of penicillins, cephalosporins, tetracyclines, erythromycins and other antibiotics;
– said at least one medium in said initial admixture includes a solvent selected from the group consisting of dimethylformamide, dimethylacetamide, dimethylsulfoxide and N-methylpyrrolidone;
– said at least one medium in said initial admixture includes a solvent selected from the group consisting of aliphatic alcohols containing 1 to 6 carbon atoms, alkyl acetates having from 1 to 6 carbon atoms in the alkyl group, aliphatic ethers containing 4 to 8 carbon atoms, aliphatic ketones containing 3 to 8 carbon atoms, aliphatic nitriles containing 1 to 6 carbon atoms and aliphatic hydrocarbons containing from 5 to 10 carbon atoms;
– said procedure is conducted at ambient temperature.

7. A method according to any of the preceding claims, wherein said procedure is characterized by the fact that the concentration of the said substance at said feed surface is periodically monitored, and the procedure is terminated when a predetermined increase in said concentration, compared with the concentration in said initial admixture, has been achieved.

8. A method according to claim 4, wherein at least one of the following conditions applies, namely:
– said crosslinked layer includes a crosslinked bromomethylated 2,6-dimethylphenylene oxide polymer which has been derivatized so as to introduce hydrophobic groups;
– said crosslinked layer includes a crosslinked bromomethylated 2,6-dimethylphenylene oxide polymer which has been derivatized so as to introduce both ionic and hydrophobic groups;
– said substrate includes at least one material selected from polyarylene oxides, polyarylene sulfones, tetrafluoropolyethylene, and other perfluoro polymers;
– said substrate includes at least one material selected from crosslinked polyacrylonitrile, polyethylene, polypropylene, other polyolefins, aromatic polyimides, glass, ceramics and carbon, and additionally at least one material selected from polyarylene oxides, polyarylene sulfones, tetrafluoropolyethylene, and other perfluoro polymers.

9. A method according to any of claims 1 to 5, wherein at least one of the following conditions applies, namely:
– said at least one substance is selected from the group consisting of enzymes, hormones, proteins and antibodies;
– said at least one medium in said initial admixture includes at least one solvent selected from the group consisting of halohydrocarbons and aromatic hydrocarbons;
– said at least one medium in said initial admixture includes at least one solvent selected from the group consisting of halohydrocarbons and aromatic hydrocarbons, together with at least one solvent selected from the group consisting of dimethylformamide, dimethylacetamide, dimethylsulfoxide and N-methylpyrrolidone, aliphatic alcohols containing 1 to 6 carbon atoms, alkyl acetates having from 1 to 6 carbon atoms in the alkyl group, aliphatic ethers containing 4 to 8 carbon atoms, aliphatic ketones containing 4 to 8 carbon atoms, aliphatic nitriles containing 1 to 6 carbon atoms and aliphatic hydrocarbons containing from 5 to 10 carbon atoms.

**10.** A method according to claim 1, wherein said solvent stable membrane is defined as in one of the following paragraphs, (I), (II) and (III), namely:

(I) said solvent stable membrane comprises a porous substrate, such as a substrate cast from a polymer, selected from copolymers and homopolymers of ethylenically unsaturated nitriles, coated with a silicone layer, which substrate has preferably been treated with a pore protector in absence of a curing agent, prior to coating with a silicone layer;

(II) said solvent stable membrane is made by a process which includes applying the following sequential steps to a porous membrane substrate which has been crosslinked and insolubilized, namely: (a) applying to the surface and pores of the substrate, a coating of reactive polymer; (b) treating the cross-linked coated substrate with a multifunctional agent; (c) treating the product of step (b) with further reactive polymer which may be the same as or different from that mentioned in step (a); and (d) treating the product of step (c) with further multifunctional agent which may be the same as or different from that mentioned in step (b);

(III) said solvent stable membrane is as defined in claim 4, and further said crosslinked layer includes at least one polymer derived from halomethylated polyphenylene oxide type monomers, the crosslinkers utilized for crosslinking the polyphenylene oxide type polymeric layer being selected from aliphatic, cycloaliphatic, araliphatic and aromatic amines, and said crosslinked layer is preferably less than 1 micron in thickness, and/or it may be ionically charged.

**11.** A method according to claim 10, wherein at least one of the following conditions applies, namely:

– said substrate is selected from microfiltration membranes and ultrafiltration membranes;

– said substrate includes at least one material selected from crosslinked polyacrylonitrile, polyarylene oxides, polyarylene sulfones, polyethylene, polypropylene, other polyolefins, tetrafluoropolyethylene, other perfluoro polymers, aromatic polyimides, glass, ceramics and carbon;

– said at least one substance is selected from the group consisting of enzymes, hormones, proteins, antibodies, $\alpha$-aminoacids, dipeptides, tripeptides, tetrapeptides and higher order peptides;

– said at least one substance is selected from the group consisting of penicillins, cephalosporins, tetracyclines, erythromycins and other antibiotics;

– said at least one medium in said initial admixture includes a solvent selected from the group consisting of dimethylformamide, dimethylacetamide, dimethylsulfoxide and N-methylpyrrolidone, aliphatic alcohols containing 1 to 6 carbon atoms, alkyl acetates having from 1 to 6 carbon atoms in the alkyl group, aliphatic ethers containing 4 to 8 carbon atoms, aliphatic ketones containing 3 to 8 carbon atoms, aliphatic nitriles containing 1 to 6 carbon atoms, aliphatic hydrocarbons containing from 5 to 10 carbon atoms, halohydrocarbons and aromatic hydrocarbons;

– said procedure is conducted at ambient temperature;

– said procedure is characterized by the fact that the concentration of the said substance at said feed surface is periodically monitored, and the procedure is terminated when a predetermined increase in said concentration, compared with the concentration in said initial admixture, has been achieved.

**12.** A solvent stable membrane which possesses simultaneously the properties of rejecting at least in part biologically active substances having a molecular weight below 1500, present in an admixture with at least one medium selected from the group consisting of organic solvent media and their mixtures with water, and of allowing at least in part the permeation of at least part said at least one medium; said membrane having been made by a process which includes applying the following sequential steps to a porous membrane substrate cast from a polymer, selected from copolymers and homopolymers of ethylenically unsaturated nitriles, which has been crosslinked and insolubilized, namely: (a) coating said crosslinked and insolubilized porous membrane substrate by use of a reactive polymer; (b) treating the cross-linked substrate with a multifunctional agent, (c) treating the product of step (b) with further reactive polymer which may be the same as or different from that mentioned in step (a); and (d) treating the product of step (c) with further multifunctional agent which may be the same as or different from that mentioned in step (b), said crosslinked and insolubilized porous membrane substrate being preferably a said crosslinked and insolubilized porous membrane substrate from polyacrylonitrile which has been heated in a basic medium at an elevated temperature, the basic medium treated substrate having been again subjected to an elevated temperature after substantial removal of excess base.